# FASCICULE DE BREVET EUROPEEN

(11) **EP 3 560 521 B1**
(45) Date de publication et mention de la délivrance du brevet: **27.03.2024**
(21) Numéro de dépôt: 18169017.3
(22) Date de dépôt: 24.04.2018
(51) Int. Cl.: A61L 2/10, A61L 2/26

(54) **DISPOSITIF DE DECONTAMINATION DE LIQUIDE TURBIDE**
DEKONTAMINIERUNGSVORRICHTUNG FÜR TURBIDFLÜSSIGKEIT
DECONTAMINATION DEVICE FOR TURBID LIQUID

(43) Date de publication de la demande: 30.10.2019
(73) Titulaire: Biosafelight, 45000 Orleans (FR)
(72) Inventeur: JOVENIAUX, Christophe, 45400 CHANTEAU (FR)

(56) Documents cités:
- WO-A1-2010/107145
- WO-A1-2017/186510
- WO-A2-01/37675
- DE-A1- 4 025 078
- DE-C- 909 292

## Description

### DOMAINE TECHNIQUE DE L'INVENTION

L'invention concerne un dispositif de décontamination d'un liquide turbide, en particulier un liquide alimentaire.

### ETAT DE LA TECHNIQUE ANTERIEURE

Les dispositifs de décontamination d'un liquide réalisent une décontamination en utilisant les rayons ultra-violets. Pour cela, comme illustré dans le document EP1255444, de tels dispositifs de décontamination comprennent une gaine comportant au moins une paroi externe et une paroi interne de sorte à délimiter un espace d'écoulement du liquide à décontaminer. Dans un espace interne radialement délimité par la paroi interne de la gaine, une source à rayons ultra-violets est disposée de sorte à irradier, à travers la paroi interne de la gaine, le liquide à décontaminer. Afin de permettre une exposition la plus longue, la paroi externe de la gaine présente une surface en regard de l'espace d'écoulement qui est spiralée : ainsi, le déplacement du liquide turbide à décontaminer s'effectue selon une hélice autour de la source à rayons ultra-violets et non plus parallèlement à l'axe de la gaine. Toutefois, il est connu que les rayons ultra-violets pénètrent difficilement au sein des liquides turbides. De ce fait, pour assurer une bonne décontamination du liquide turbide, il est nécessaire d'avoir un écoulement dudit liquide qui soit turbulent. Cela implique, dans le cas des dispositifs décrits dans l'art antérieur, des vitesses de déplacement élevées et donc des pressions importantes impliquant des installations industrielles conséquentes. DE 909 292 C divulgue par exemple un dispositif de décontamination dans lequel le liquide à décontaminer circule dans une gaine autour d'une source émettant des rayons ultra-violets. Ce document divulgue des parois ondulées mais ne fournit aucun indice concernant des ondulations asymétriques. WO 2010/107145 A1 présente un dispositif de désinfection d'eau comprenant une gaine où circule l'eau et où se trouve une source UV. La paroi externe de la gaine est ondulée afin d'induire un régime turbulent, cependant ce document ne mentionne pas de gaine ondulée asymétrique ni la nécessité d'augmenter la turbulence du fluide.

### EXPOSE DE L'INVENTION

Un but de l'invention est de fournir un dispositif de décontamination qui permette une décontamination optimale de liquide turbide sans pour autant nécessiter des vitesses de déplacement élevées pour obtenir un écoulement turbulent nécessaire.

A cette fin, il est prévu, selon l'invention, un dispositif de décontamination d'un liquide turbide, notamment de type alimentaire, tel que défini dans les revendications.

Ainsi, le fait d'avoir une surface cylindrique en regard de l'espace annulaire de circulation présentant un profil le long d'une génératrice de la surface cylindrique comprenant une succession de formes concaves et convexes alternativement permet d'obtenir simplement et facilement un écoulement turbulent au sein de l'espace de circulation annulaire avec des vitesse d'écoulement faible et donc des pressions moins élevés ce qui permet de réduire l'installation industrielle de dispositifs de décontamination d'un liquide turbide ainsi équipés.

Avantageusement, mais facultativement, le dispositif de décontamination d'un liquide turbide selon l'invention présente au moins l'une des caractéristiques techniques suivantes :
- le profil comporte au moins un tronçon de forme de vague ;
- la surface cylindrique est au moins en partie de forme annelée ;
- la paroi externe est réalisée en acier inoxydable ;
- la paroi interne est réalisée en un matériau laissant passer les rayons ultra-violets, notamment en quartz ; et
- la source de rayons ultra-violets comporte des diodes électroluminescentes.

### BREVE DESCRIPTION DES FIGURES

D'autres caractéristiques et avantages de l'invention ressortiront à la lecture de la description qui suit d'un mode de réalisation de l'invention. Aux dessins annexés :
- la figure 1 est une vue de face d'un dispositif de décontamination selon l'invention ;
- la figure 2 est une vue de dessus en coupe selon II-II du dispositif de la figure 1 ;
- la figure 3 est une vue de face d'un exemple illustratif d'une paroi externe de la gaine du dispositif de décontamination
- la figure 4 est une vue de face d'un mode de réalisation d'une paroi externe de la gaine du dispositif de décontamination selon l'invention ;
- la figure 5 est une vue de détail V en coupe de la paroi externe de la figure 4 ; et,
- la figure 6 est une vue tridimensionnelle de la paroi externe de la figure 4.

Pour plus de clarté, les éléments identiques ou similaires sont repérés par des signes de référence identiques sur l'ensemble des figures.

### DESCRIPTION DETAILLEE D'UN MODE DE REALISATION

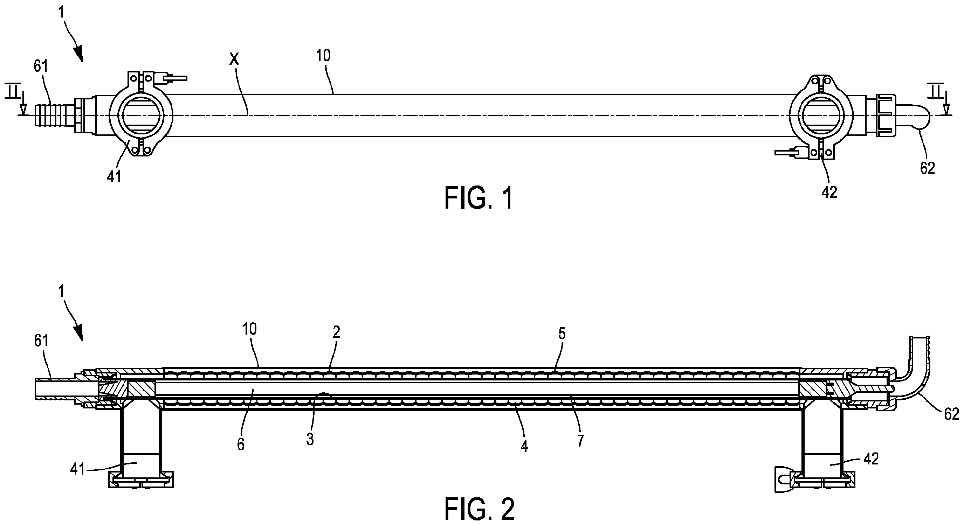

En référence aux figures 1 et 2, nous allons décrire un dispositif de décontamination d'un liquide turbide 1 selon l'invention.

Le dispositif de décontamination d'un liquide turbide 1 selon l'invention comporte un carter 10 de forme allongée globalement cylindrique d'axe, un axe longitudinal X. Le carter 10 entoure une gaine 5, ainsi qu'une source de rayons ultra-violets 6 s'étendant longitudinalement selon l'axe longitudinal X et coaxialement au carter 10 et à la gaine 5. Le carter 10 comporte, à chacune de ses extrémités, des moyens d'accès et de connexion 61,62 à la source de rayons ultra-violets 6. Par exemple, la source de rayons ultra-violets 6 est une lampe au mercure connue en soi, ou encore des diodes électroluminescentes. Bien entendu, d'autres systèmes émettant des rayons ultra-violets peuvent être utilisés dans le dispositif de décontamination d'un liquide turbide 1 selon l'invention.

La gaine 5 est de forme générale cylindrique d'axe longitudinal X, en particulier de forme générale cylindrique de révolution. Elle comporte une paroi externe 2 et une paroi interne 3 s'étendant en regard et à distance l'une de l'autre et coaxiales l'une par rapport à l'autre. Les deux parois externe 2 et interne 3 délimitent, entre elles, un espace de circulation 4 de forme annulaire dans lequel est destiné à circuler le liquide à décontaminer. D'autre part, la paroi interne 3 délimite un espace central 7, radialement interne, dans lequel est positionnée la source de rayons ultra-violets 6. Ainsi, l'espace central 7 est coaxial à et entouré par l'espace annulaire de circulation 4 du liquide à décontaminer.

**A** chacune des extrémités selon l'axe longitudinal X de la gaine 5, l'espace annulaire de circulation 4 du liquide à décontaminer est fluidiquement connecté, respectivement à une entrée 41 du liquide à décontaminer et à une sortie 42 du liquide alors décontaminé. Ici, les entrée 41 et sortie 42 sont orientées de manière radiale et centrifuge.

La paroi interne 3 est réalisée en un matériau laissant passer les rayons ultra-violets émis par la source de rayons ultra-violets 6 de sorte que lesdits rayons ultra-violets puissent diffuser dans l'espace annulaire de circulation 4 lors d'un fonctionnement du dispositif de décontamination d'un liquide turbide 1 selon l'invention. Par exemple, le matériau utilisé est du quartz.

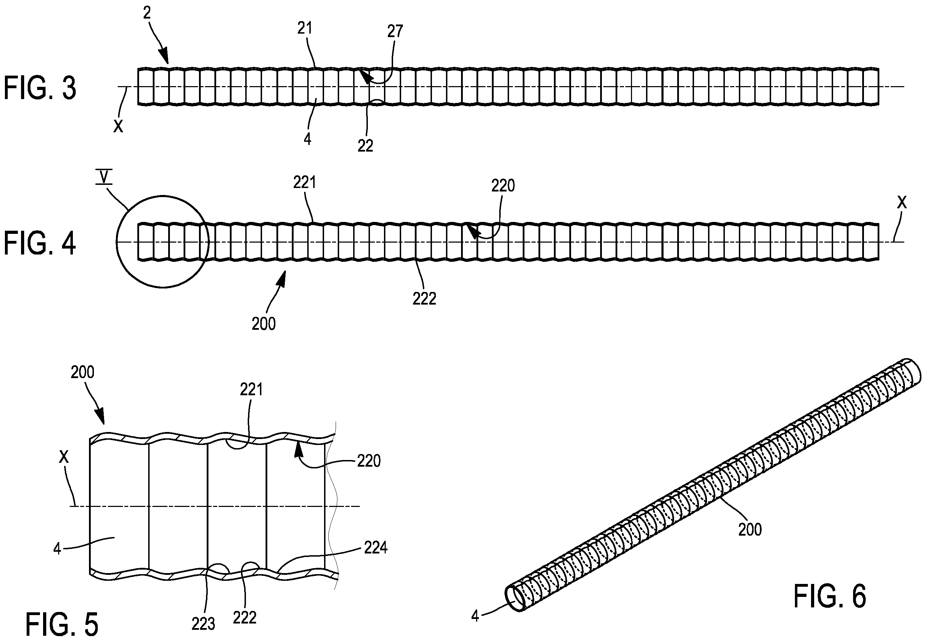

En référence à la figure 3, nous allons décrire plus en détail un exemple illustratif de la paroi externe 2 pour un dispositif de décontamination d'un liquide turbide 1. La paroi externe 2 est réalisée en un matériau inoxydable par exemple, tel que de l'acier inoxydable de grade alimentaire 316L. La paroi externe 2 comporte une surface cylindrique 27 qui s'étend en regard de l'espace annulaire de circulation 4 du liquide à décontaminer, d'une part, et, d'autre part, en regard et à distance de la paroi interne 3. La surface cylindrique 27 présente un profil le long d'une génératrice de ladite surface cylindrique 27 qui est accidenté. Le profil le long d'une génératrice de la surface cylindrique 27 comprend une succession de formes concaves 21 et convexes 22 alternativement. Il est à noter que la concavité et la convexité des formes s'établit par rapport à l'espace annulaire de circulation 4. Illustré à la figure 2, le profil le long d'une génératrice de la surface cylindrique 27 comporte des formes concaves 21 qui sont des arcs de cercles et des formes convexes 22 qui sont des pointes saillantes dans l'espace annulaire de circulation 4, chacune des pointes saillantes étant obtenue par la jonction de deux arcs de cercle formant les formes concaves 21 adjacents. Ainsi la surface cylindrique 27 présente une forme annelée. En variante de réalisation, les arcs de cercle sont remplacés par des arcs d'ellipse.

Dans un mode de réalisation de la paroi externe 200 du dispositif de décontamination d'un liquide turbide 1 selon l'invention, illustré aux figures 4 à 6, la paroi externe 200 comporte, comme précédemment, une surface cylindrique 270 qui s'étend en regard de l'espace annulaire de circulation 4 du liquide à décontaminer, d'une part, et, d'autre part, en regard et à distance de la paroi interne 3. La surface cylindrique 270 présente, ici aussi, un profil le long d'une génératrice de ladite surface cylindrique 270 qui est accidenté. De nouveaux, le profil le long d'une génératrice de la surface cylindrique 270 comprend une succession de formes concaves 221 et convexes 222 alternativement. Illustré au figures 4 et 5, le profil le long d'une génératrice de la surface cylindrique 270 comporte une succession de vagues comportant des creux formant les formes concaves 221 et des sommets formant les formes convexes 222. Alternativement le long du profil, un creux 221 et un sommet 222 sont reliés par une pente longue 223 et une pente courte 224. En variante de réalisation, la succession de vagues forme une sinusoïde le long du profil le long d'une génératrice de la surface cylindrique 270.

Dans un exemple illustratif supplémentaire, le profil le long d'une génératrice de la surface cylindrique 27, 270 comprend une succession d'arcs de cercle alternativement concaves 21 et convexes.

La forme du profil le long d'une génératrice de la surface cylindrique 27, 270 est choisie en fonction du liquide turbide qui va être décontaminé par le dispositif de décontamination d'un liquide turbide 1 selon l'invention. Les paramètres turbidité et de viscosité du liquide à décontaminer permettent à la personne de l'art de choisir la forme du profil idoine de sorte à optimiser la décontamination du liquide turbide sans pour autant nécessiter des vitesses de déplacement élevées pour obtenir l'écoulement turbulent nécessaire dans l'espace annulaire 4 du dispositif de décontamination d'un liquide turbide 1 selon l'invention.

Bien entendu, il est possible d'apporter à l'invention de nombreuse modification sans pour autant sortir du cadre de celle-ci. à condition de rester dans le cadre des revendications.

## Revendications

1. Dispositif de décontamination d'un liquide turbide (1), notamment de type alimentaire, comprenant une gaine (5) s'étendant longitudinalement selon un axe X, comportant une paroi externe (2 ;200) et une paroi interne (3) coaxiales l'une par rapport à l'autre et délimitant un espace annulaire de circulation (4) du liquide à décontaminer, une entrée (41) et une sortie (42) du liquide à décontaminer fluidiquement connectés à l'espace annulaire de circulation (4), et une source (6) de rayons ultra-violets positionnée coaxialement à l'espace annulaire de circulation, la paroi externe comportant une surface cylindrique (27;270) en regard de l'espace annulaire de circulation présentant un profil le long d'une génératrice de la surface cylindrique comprenant une succession de formes concaves (21;221) et convexes (22,222) alternativement, la paroi interne (3) est réalisée dans un matériau laissant passer les rayons ultra-violets, la source de rayons ultra-violet est positionnée dans un espace central (7) délimité par la paroi interne (3) de la gaine (5), et l'entrée (41) et la sortie (42) sont orientée de manière radiale et centrifuge,
**caractérisé en ce que**
le profil le long de la génératrice de la surface cylindrique (270) comporte une succession de vagues comportant des creux formant les formes concaves (221) et des sommets formant les formes convexes (222) et dans lequel chaque creux (221) est relié au creux qui le succède par la succession d'une pente courte (223), d'un des sommets (222) et d'une pente longue (224).

2. Dispositif selon l'une des revendications 1 à 3, **caractérisé en ce que** la surface cylindrique (27) est au moins en partie de forme annelée.

3. Dispositif selon l'une des revendications 1 à 4, **caractérisé en ce que** la paroi externe est réalisée en acier inoxydable.

4. Dispositif selon l'une des revendications 1 à 5, **caractérisé en ce que** la paroi interne est réalisée en un matériau laissant passer les rayons ultra-violets, notamment en quartz.

5. Dispositif selon l'une des revendications 1 à 6, **caractérisé en ce que** la source de rayons ultra-violets comporte des diodes électroluminescentes.

## Patentansprüche

1. Vorrichtung zur Dekontaminierung einer trüben Flüssigkeit (1), insbesondere aus dem Lebensmittelbereich, umfassend einen Mantel (5), der sich in Längsrichtung entlang einer Achse X erstreckt, umfassend eine Außenwand (2; 200) und eine Innenwand (3), die koaxial zueinander sind und einen ringförmigen Zirkulationsraum (4) für die zu dekontaminierende Flüssigkeit begrenzen, einen Einlass (41) und einen Auslass (42) für die zu dekontaminierende Flüssigkeit, die fluidtechnisch mit dem ringförmigen Zirkulationsraum (4) verbunden sind, und eine Quelle (6) ultravioletter Strahlen, die koaxial zu dem ringförmigen Zirkulationsraum positioniert ist, wobei die Außenwand eine zylindrische Oberfläche (27; 270), die dem ringförmigen Zirkulationsraum zugewandt ist und ein Profil entlang einer Mantellinie der zylindrischen Oberfläche aufweist, das eine Abfolge von abwechselnd konkaven (21; 221) und konvexen (22; 222) Formen umfasst, die Innenwand (3)
besteht aus einem für ultraviolette Strahlen durchlässigen Material, die Quelle ultravioletter Strahlen ist in einem zentralen Bereich (7) positioniert, der von der Innenwand (3) des Mantels (5) begrenzt wird, und der Einlass (41) und der Auslass (42) sind radial und zentrifugal ausgerichtet, **dadurch gekennzeichnet, dass**
das Profil entlang der Mantellinie der zylindrischen Oberfläche (270) eine wellenartige Abfolge von Vertiefungen, die die konkaven Formen (221) bilden, und Scheiteln, die die konvexen Formen (222) bilden, umfasst und wobei jede Vertiefung (221) mit der nachfolgenden Vertiefung über die Abfolge einer kurzen Steigung (223), einem der Scheitel (222) und einer langen Steigung (224) verbunden ist.

2. Vorrichtung nach einem der Ansprüche 1 bis 3, **dadurch gekennzeichnet, dass** die zylindrische Oberfläche (27) zumindest teilweise eine geriffelte Form aufweist.

3. Vorrichtung nach einem der Ansprüche 1 bis 4, **dadurch gekennzeichnet, dass** die Außenwand aus rostfreiem Stahl besteht.

4. Vorrichtung nach einem der Ansprüche 1 bis 5, **dadurch gekennzeichnet, dass** die Innenwand aus einem Material besteht, das ultraviolette Strahlen durchlässt, insbesondere aus Quarz.

5. Vorrichtung nach einem der Ansprüche 1 bis 6, **dadurch gekennzeichnet, dass** die Quelle ultravioletter Strahlen Leuchtdioden umfasst.

## Claims

1. Device for decontaminating a turbid liquid (1), in particular of the liquid food type, comprising a sheath (5) extending longitudinally along an axis X, comprising an outer wall (2; 200) and an inner wall (3) that are coaxial with one another and delimiting an annular circulation space (4) for circulating the liquid to be decontaminated, an inlet (41) and an outlet (42) for the liquid to be decontaminated, which inlet and outlet are fluidly connected to the annular circulation space (4), and a source (6) of ultraviolet rays positioned coaxially with the annular circulation space, the outer wall comprising a cylindrical surface (27; 270) facing the annular circulation space and having a profile along a generatrix of the cylindrical surface that includes an alternating succession of concave shapes (21; 221) and convex shapes (22; 222), the internal wall (3) being made of a material that allows ultraviolet rays to pass therethrough, the source of ultraviolet rays being positioned in a central space (7) delimited by the inner wall (3) of the sheath (5), and the inlet (41) and the outlet (42) being oriented radially and centrifugally, **characterised in that** the profile along the generatrix of the cylindrical surface (270) comprises a succession of waves comprising hollows forming the concave shapes (221) and peaks forming the convex shapes (222) and in which each hollow (221) is connected to the following hollow by the succession of a short slope (223), one of the peaks (222) and a long slope (224).

2. Device according to one of claims 1 to 3, **characterised in that** the cylindrical surface (27) is at least partially ringed.

3. Device according to one of claims 1 to 4, **characterised in that** the outer wall is made of stainless steel.

4. Device according to one of claims 1 to 5, **characterised in that** the inner wall is made of a material which allows ultraviolet rays to pass therethrough, and is in particular made of quartz.

5. Device according to one of claims 1 to 6, **characterised in that** the source of ultraviolet rays comprises light-emitting diodes.
